# EUROPEAN PATENT APPLICATION

(11) **EP 3 763 396 A1**
(43) Date of publication of application: **13.01.2021**
(21) Application number: 20185076.5
(22) Date of filing: 09.07.2020
(51) Int. Cl.: A61L 15/26, A61L 15/28, A61L 15/60, A61L 26/00

(54) **METHOD FOR COLOR MAINTENANCE AND ENHANCEMENT OF TATTOO**

(30) Priority: 10.07.2019 TW 108124358
(71) Applicant: MLK Bioscience Co., Ltd., Taoyuan City, Taiwan 330 (TW)
(72) Inventor: WANG, WAN-TING, 231 New Taipei City (TW); Su, Tian-Yu, 231 New Taipei City (TW)
(74) Representative: Lang, Christian

(57) **Abstract**

The present invention is a waterproof and air-permeable moist dressing which moisturizes and enhances color for aftercare of fresh tattoo. Therefore, the present invention is capable of providing professional and medical repair, protection, isolation, and absorbing wound exudates and tattoo inks after tattooing to improve the aesthetical beauty of the tattoo.

## Description

### FIELD OF THE INVENTION

The present invention is a waterproof and air-permeable moist dressing which moisturizes and enhances color for aftercare of fresh tattoo. Therefore, the present invention is capable of providing professional and medical repair, protection, isolation, and absorption of wound exudates and tattoo inks after tattooing to improve the aesthetical beauty of the tattoo.

### BACKGROUND OF THE INVENTION

In recent years, tattoo art has become more and more popular. As the number of people who have tattoos increases, more and more people start to pay notice the unmet neet of tattoo wound repair. In the past, the main principle of tattoo aftercare was to keep the wounds in a ventilated and air-permeable conditioon. Accordingly, only vaseline or tattoo ointment was applied for moisturization without covering the wounds with any dressings.

However, the tattoo wounds are not completely isolated by applying vaseline or tattoo ointment only and can easily get touched with clothings, resulting in inconvenience and discomfort, even conditions requiring constant medication applications. Tattoo wounds are open wounds. Without isolation, when contacting with water (bathing or showering) or bacteria from the environment or clothings, the wounds can be infected, leading to inflammation or oozing of tattoo inks. Furthermore, without an isolation layer, after scar formation in the tattoo wounds, the scars can easily be torn off by conforters, bed sheets, towels, and clothings, causing the tattoo wounds to be opened up again, leading to infection or discoloration. In addition, when the tattoo wounds are not isolated, patients tend to scratch the wounds due to itching caused by dryness, which also leads to infection or discoloration. This loss of ink negatively affects the technical quality and visual aesthetics of tattoos.

To protect the wounds caused by a fresh tattoo, recent tattoo artists use a petrochemical plastic (for example a plastic wrap) as an occlusive layer and auxiliary dressing, which is usually not of medical-grade quality. The household plastic wrap material does not absorb wound exudates, and is wrapped around the skin to seal the wound exudates and oozed inks inside so as to avoid contacts of the wound exudates and inks with clothings after tattooing, and to reduce frictions at the same time.

However, the plastic wrap is not an air-permeable material, which can easily lead to wound fester due to impermeability and excessive humidity. Therefore, the plastic wrap is only used as an occlusive layer within three hours after a fresh tattoo, and must be removed after three hours. In addition to keeping the wounds in a condition that is impermeable, humid, discomfortable, and prone to infections, the plastic wrap is easy to fall off.

In the past, tattooing were not regarded as a medical action, and an open wound treatment after formal surgeries was hardly considered for tattoo wounds, thus no dressings had ever been used for protection, resulting in a risk of tattoo wound infection. Discoloration, dullness, scarring or daily life inconvenience caused by wound healing problems and ink loss are highly undesirable.

In order to overcome these problems, a hydrogel dressing for tattoos has been developed in recent years, for example, the US Patent Publication No. US20140200196A1, the US Patent No. US09649330B2, and the PCT Publication No. WO2014145714A1, disclose a transparent film layer formed by combining an alginate hydrogel with a mineral-containing spray solution for wound isolation, stopping bleeding and wound repairs. Since hydrogel is in a gel-like form, the structural strength of the hydrogel is insufficient to buffer physical impacts and frictions of the wound.

In addition, these patent applications involve two materials, one is an alginate hydrogel, the other is a mineral-containing spray solution. The role of hydrogel is to promote wound repairs based on the principle of wound moisturization and treatment, and to promote hemostasis of the wounds through hemostatic factors contained in the hydrogel. However, because the outer film of the hydrogel is not waterproof, the hydrogel falls off due to dryness caused by water evaporation, and cannot be applied to wounds with no exudate. Therefore, similar to the plastic wrap, the hydrogel dressing can only be used within three hours after completion of a tattoo, and is not suitable for mintenance and repair after three hours.

The role of the film formed by the mineral-containing spray solution is to isolate the wound to prevent infection. However, the film is not waterproof and does not have sufficient adhesion. Since it is not waterproof, the wounds of a fresh tattoo cannot be in contact with water, for example, bathing, cleaning, doing outdoor activities in rainy days ... etc., otherwise the dressing falls off due to swelling of the hydrogel. Due to insufficient adhesion, the patient must avoid frictions between the dressing at the wound site and the clothing, and rubbing or slapping the wounds is no longer allowed when feeling itchy, causing unnecessary pain and inconvenience to daily activities.

Furthermore, the patent involve two steps, a first step of applying a thin layer of hydrogel first, and a second step of spraying a mineral-containing spray solution at a specific distance so as to form a film on the surface of the hydrogel after spray coagulation, showing that the process is complicated and troublesome. If it can be replaced with a sheet material, the steps and precautions can be simplified to reduce the complexity and troublesomeness.

Therefore, the problem that requires a solution in the field is how to enhance maintence, repair and color enchanement of fresh tattoos.

### DETAILED DESCRIPTION OF THE INVENTION

In order to solve the aforementioned problems, there is an urgent need to develop a method that can protect, isolate, absorb wound exudates and oozing of tattoo inks, and enhance tattoo color and visual art. The present invention provides a moist dressing, which comprises waterproofing and air-permeable, isolating, protecting and buffering effect against external environmental stimulation, keeping the skin moist, maintaining the artistic beauty of tattoo patterns.

The term "a" or "an" as used herein is to describe elements and ingredients of the present invention. This term is only for convenience of description and the basic idea of the present invention. The description should be understood as comprising one or at least one, and unless otherwise explicitly indicated by the context, singular terms include pluralities and plural terms include the singular. When used in conjunction with the word "comprising" in a claim, the term "a" or "an" may mean one or more than one.

The present invention provides a moist dressing, wherein the form of the moist dressing is a solid sheet-like dressing or a liquid spray-on dressing, and the solid sheet-like dressing comprises a transparent film dressing, a hydrogel dressing, a hydrocolloid dressing, a hydrofiber dressing, an alginate dressing, a silicone dressing or a chitosan dressing.

The "moist dressing" of the present invention is a dressing that can provide tattoo patterns a more humid environment after tattooing, passively creating an environment that is beneficial to the maintenance and repair of the tattoo patterns after tattooing in order to maintain the tattoo patterns and bright colors.

In one embodiment of the present invention, the moist dressing has waterproof and air-permeable function. In a preferred embodiment of the present invention, the moist dressing comprises a waterproof and air-permeable film.

In one embodiment of the present invention, the moist dressing comprises a soothing factor, a retouching factor or a combination thereof. The soothing factor or the retouching factor can be optionally present in the moist dressing, wherein the soothing factor or the retouching factor are maintenance components or combinations thereof capable of enhancing comfort, color enhancement and color maintenance.

In one embodiment of the present invention, the soothing factor is capable of enhancing comfort, such as avoiding scars, stopping itches, soothing and calming, reducing wound odors, etc. Thus in a preferred embodiment of the present invention, the soothing factor is selected from the group consisting of collagen, hyaluronic acid, glycerin, neuroamide, gelatin, growth factors, cytokines, antibacterial peptides, chitosan, diclofenac sodium, indomethacin, menthol, camphor, thymol, tea tree oil, clove oil, borneol, capsaicin, nicotinic acid benzyl ester, eucalyptus oil, curcumin, musk, cinnamon, angelica, frankincense, and derivatives thereof.

In one embodiment of the present invention, the retouching factor is capable of moisturizing and maintaining colors, such as retaining moisture, brightening colors, retouching colors, whitening, etc. Therefore, in a more preferred embodiment of the present invention, the retouching factor is selected from the group consisting of glycyrretinic acid, salvia miltiorrhiza, allantoin, aloe, arbutin, vitamin C (ascorbyl acid), salicylic acid, ellagic acid, kojic acid, chamomile extract (chamomile ET), tranexamic acid, 5,5'-dipropyl-biphenyl-2,2'-diol and deratives thereof.

In one embodiment of the present invention, the weight percentage of the soothing factor or the retouching factor is from 0.01% to 10% by weight of the total components of the moist dressing.

In one embodiment of the present invention, the retouching factor comprises potassium methoxysalicylate, aloe, or a combination thereof. In a preferred embodiment of the present invention, the retouching factor has a moisturizing and color maintaining effect, comprising 0.5% of potassium methoxysalicylate, 2% of aloe or a combination thereof.

In one embodiment of the present invention, the retouching factor comprises arbutin, and the soothing factor comprises hyaluronic acid. In a preferred embodiment of the present invention, the retouching factor comprises 0.5 g of arbutin, and the soothing factor comprises 0.1 g of hyaluronic acid.

The present invention further provides a waterproof and air-permeable moist dressing, which comprises a waterproof and air-permeable film which is provided with an adhesive surface; and a hydrogel which is provided with a first surface and a secod surface, wherein the first surface is used for being bonded to the adhesive surface of the waterproof and air-permeable film, and the second surface is used for being adhered to a tattoo pattern of the skin of a subject; wherein the waterproof and air-permeable moist dressing is adhered onto the tattoo pattern so as to maintain the bright color of the tattoo pattern and to provide a retouching effect.

In another embodiment of the present invention, the hydrogel has a waterproof and air-permeable function.

In one embodiment of the present invention, the hydrogel has a maintaining effect such as comfort improving, protecting and isolating, structure buffering, water locking and color retouching effect. In a preferred embodiment of the present invention, the hydrogel comprises a soothing factor, a retouching factor or a combination thereof.

In one embodiment of the present invention, the soothing factor is selected from the group consisting of collagen, hyaluronic acid, glycerin, neuroamide, gelatin, growth factors, cytokines, antibacterial peptides, chitosan , diclofenac sodium, indomethacin, menthol, camphor, thymol, tea tree oil, clove oil, borneol, capsaicin, nicotinic acid benzyl ester, eucalyptus oil, curcumin, musk, cinnamon, angelica, frankincense and derivatives thereof.

In another embodiment of the present invention, the retouching factor is selected from the group consisting of glycyrretinic acid, salvia miltiorrhiza, allantoin, aloe, arbutin and vitamin C (ascorbyl acid), salicylic acid, ellagic acid, kojic acid, chamomile extract (Chamomile ET), tranexamic acid, 5,5'-dipropyl biphenyl-2,2'-diol and derivatives thereof.

In one embodiment of the present invention, the weight percentage of the soothing factor or the retouching factor is from 0.01% to 10% by weight of the total components of the hydrogel.

In one embodiment of the present invention, the retouching factor comprises potassium methoxysalicylate, aloe, or a combination thereof. In a preferred embodiment of the present invention, the retouching factor has a moisturizing and color maintaining effect, comprising 0.5% of potassium methoxysalicylate, 2% of aloe or a combination thereof.

In one embodiment of the present invention, the retouching factor comprises arbutin, and the soothing factor comprises hyaluronic acid.

The present invention further provides a method for color maintenance and color enhancement of a fresh tattoo pattern, comprising: (a) tattooing a fresh pattern on the skin of a subject; and (b) covering a moist dressing on the fresh pattern, wherein the moist dressing is used to maintain bright color of the pattern and to provide a retouching effect, and comprises a solid sheet-like dressing or a liquid spray-on film dressing, wherein the solid sheet-like dressing comprises a transparent film dressing, a hydrogel dressing, a hydrocolloid dressing, a hydrofiber dressing, an alginate dressing, a silicone dressing or a chitosan dressing.

In one embodiment of the present invention, the subject is a mammal. In a preferred embodiment, the subject is human.

In one embodiment of the present invention, the step (a) further comprises covering the moist dressing on the pattern within 2 hours after tattooing. In a preferred embodiment of the present invention, the step (a) further comprises covering the moistdressing on the pattern within 1 hour after tattooing. In a more preferred embodiment of the present invention, the step (a) further comprises covering the moist dressing on the pattern within 30 minutes after tattooing.

In another embodiment of the present invention, the step (b) further comprises a step (b1), wherein the step (b1) comprises removing the moist dressing 48 hours after covering the moist dressing on the pattern, and then covering a new moist dressing on the pattern. In a more preferred embodiment of the present invention, the step (b) further comprises a step (b1), wherein the step (b1) comprises removing the moist dressing 24 hours after covering the moist dressing on the pattern, and then covering a new moist dressing on the pattern.

In one embodiment of the present invention, the step (b1) is repetitively performed six times. In a preferred embodiment of the present invention, the step (b1) is repetitively performed four times. In a more preferred embodiment of the present invention, the step (b1) is repeatitively performed twice.

In another embodiment of the present invention, the moist dressing comprises a soothing factor, a retouching factor, or a combination thereof.

In one embodiment of the present invention, the soothing factor is selected from the group consisting of collagen, hyaluronic acid, glycerin, neuroamide, gelatin, growth factors, cytokines, antibacterial peptides, chitosan, diclofenac sodium, indomethacin, menthol, camphor, thymol, tea tree oil, clove oil, borneol, capsaicin, nicotinic acid benzyl ester, eucalyptus oil, curcumin, musk, cinnamon, angelica, frankincense and derivatives thereof.

In another embodiment of the present invention, the retouching factor is selected from the group consisting of glycyrretinic acid, salvia miltiorrhiza, allantoin, aloe, arbutin, vitamin C (ascorbyl acid), salicylic acid, ellagic acid, kojic acid, chamomile extract (chamomile ET), tranexamic acid, 5,5'-dipropyl-biphenyl-2,2'-diol and derivatives thereof.

In one embodiment of the present invention, the weight percentage of the soothing factor or the retouching factor is from 0.01% to 10% by weight of the total components of the moist dressing.

In one embodiment of the present invention, the retouching factor comprises potassium methoxysalicylate, aloe or a combination thereof. In a preferred embodiment of the present invention, the retouching factor has a water retaining and color maintaining effect, comprising 0.5% of potassium methoxysalicylate, 0.2% of aloe or a combination thereof.

In one embodiment of the present invention, the retouching factor comprises arbutin, and the soothing factor comprises hyaluronic acid.

In one embodiment of the present invention, the moist dressing has a waterproof and air-permeable function. In a preferred embodiment of the present invention, the moist dressing comprises a waterproof and air-permeable film.

The present invention provides a composition for preparing a pharmaceutical composition for maintaining and reparing patterns and colors of a fresh tattoo, wherein the composition comprises a transparent film, hydrogel, hydrocolloid, hydrofiber, alginate, silicone, chitosan, a liquid spray-on film or a combination thereof, and the pharmaceutical composition is a moist dressing, wherein the moist dressing is used for covering a fresh totttoo after tattooing to maintain the patterns and colors of the tattoo and also to provide a retouching effect.

In one embodiment of the present invention, the form of the moist dressing comprises a solid sheet-like dressing or a liquid spray-on film dressing, wherein the solid sheet-like dressing comprises a transparent film dressing, a hydrogel dressing, a hydrocolloid dressing, a hydrofiber dressing, an alginate dressing, a silicone dressing or a chitosan dressing.

In one embodiment of the present invention, the composition is hydrogel, and the moist dressing is a solid sheet-like hydrogel dressing.

In one embodiment of the present invention, the moist dressing has a waterproof and air-permeable function. In a preferred embodiment of the present invention, the moist dressing comprises a waterproof and air-permeable film.

The present invention is to provide a tattoo-specific dressing, which allows a fresh tattoo to have medical-grade of maintenance and repair and professional artistic aesthetic appeal; protects and isolates the tattoo so as not to be infected, not to be subjected to adhersion to and friction with daily contact objects such as clothings, comforters, etc., is waterproof and air-permeable to reduce wound fester, inconveniences in bathing and ink dilution and dissipation, moisturizes and maintains colors so as to enhance pigments adhesion, thereby perfectly displaying visual aesthetics of tattoo art.

### BRIEF DESCRIPTION OF THE DRAWINGS

Figure 1 shows a schematic diagram of applying a soothing factor or a retouching factor to a tattoo site.
Figure 2 shows a schematic diagram of applying a moist dressing on a tattoo area after applying a soothing factor or a retouching factor.
Figure 3 shows a schematic diagram of a side view of the structure of a waterproof and air-permeable moist dressing.

### EXAMPLES

Several examples of the operation mode of the instant application are provided, and described and illustrated in conjunction with the drawings for review and reference. For easy explanation, the drawings of the present invention are not necessarily made according to actual proportions. The proportions and locations of the features in the drawings are not intended to limit the scope of the claims of the present invention.

The following examples are non-limiting and represent only several aspects and features of the present invention.

### Example 1

Clean and cold water was used to clean and then a gause was used to gently remove blood, exudates, inks and other substances from a fresh tattoo, a hydrocolloid dressing (moist dressing 10) having a size at least 0.5 cm bigger around the tattoo was used and gently adhered onto the tattoo , the dressing was replaced every 0.5-2 days (the frequency of replacement was adjusted depending on the amount of blood and wound exudates), and aforementioned steps were repeated until the wound healed.

### Example 2

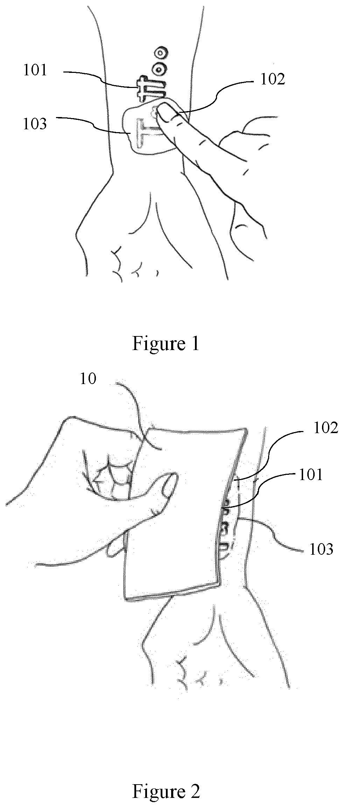

Please refer to Figures 1 and 2, clean and cold water was used to clean, then a gauze was used to gently remove blood, exudates, inks and other substances from a fresh tattoo, a retouching factor 103 containing a solution of 0.5% of potassium methoxysalicylate or 2% of aloe was applied to the tattoo 101, waited until it was in a slightly wet state, a hydrocolloid dressing (moist dressing 10) having a size at least 0.5 cm bigger around the tattoo 101 was gently adhered to the tattoo 101, the dressing was replaced every 0.5-2 days (the frequency of replacement was adjusted depending on the amount of blood and exudates), and the aforementioned steps were repeated.

Please refer to Figure 2, a moist dressing 10 containing a retouching factor 103, which was 0.5% of potassium nethoxysalicylate or 2% of aloe, was provided in this Example, a tattoo 101 was on the skin of a human body, and the moist dressing 10 was covered on the tattoo 101.

### Example 3

A hydrogel 202 having a water content of more than 50% was combined with a waterproof and air-permeable film 201 to form a waterproof and air-permeable moist dressing 20 having a self-adhesive periphery; clean and cold water was used to clean and a gauze was used to gently remove blood, exudates, inks and other substances from a fresh tattoo, the waterproof and air-permeable moist dressing 20 was adhered to a fresh tattoo 101, the dressing was replaced every 0.5-2 days (the frequency of replacement was adjusted depending on the amount of blood and wound exudates).

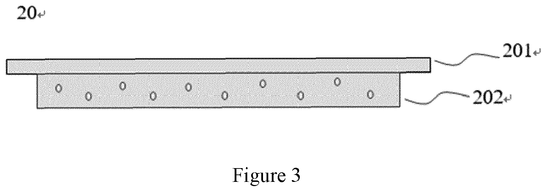

Please refer to Figure 3, which represents a schematic diagram of a side view of the structure of a waterproof and air-permeable moist dressing 20 of the present invention. The waterproof and air-permeable moist dressing 20 comprised a waterproof and air-permeable film 201 and a hydrogel 202, wherein the waterproof and air-permeable moist dressing 20 had a two-sided structure, one of which was an adhesive surface (adhesive surface is not shown), an adhesive was provided on the adhesive surface, and the adhesive surface was bonded with the hydrogel 202, so that the waterproof and air-permeable film 201 and the hydrogel 202 constituted the waterproof and air-permeable moist dressing 20. In addition, the surface area of the waterproof and air-permeable film 201 was greater than the surface area of the hydrogel 202, so that the adhesive surface of the waterproof and air-permeable film 201 had an adhesive area in excess of the area adhered to the hydrogel 202, thereby allowing the waterproof and air- permeable moist dressing 20 to be adhered to the skin.

### Example 4

A soothing factor 102, 0.1g of hyaluronic acid, and a retouching factor 103, 0.5g of arbutin, were added to 99g of hydrogel to form an arbutin hydrogel having a water content greater than 50% and 0.1% of hyaluronic acid. The hydrogel having arbutin/hyaluronic acid was combined with the waterproof and air-permeable film 201 of Example 3 to form a waterproof and air-permeable moist dressing 20 having a self-adhesive perphery; clean and cold water was used to clean and a gauze was used to gently remove blood, exudates, inks and other substances from a fresh tattoo, the waterproof and air-permeable moist dressing 20 was gently adhered onto the fresh tattoo, the dressing was replaced every 0.5-2 days (the frequency of replacement was adjusted depending on the amount of blood and exudates).

5 people after having a fresh tattoo were recruited and assigned to each group of the aforementioned different embodiments and the plastic wrap commonly used in the tattoo industry (as a comparative example) for maintenance and repair tests, indicators for tattoo maintenance and repair were measured, and the test results are shown in Table 1 as below.

Table 1. Test results of 20 subjects having a fresh tattoo according to the embodiments and comparative examples described in the instant application (condition classification: ★ ★ ★ best, ★★ ☆ better, ★★ good, ★ ☆ normal, ★ poor, ☆ nearly ineffective, — ineffective)

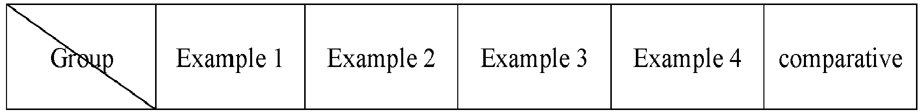

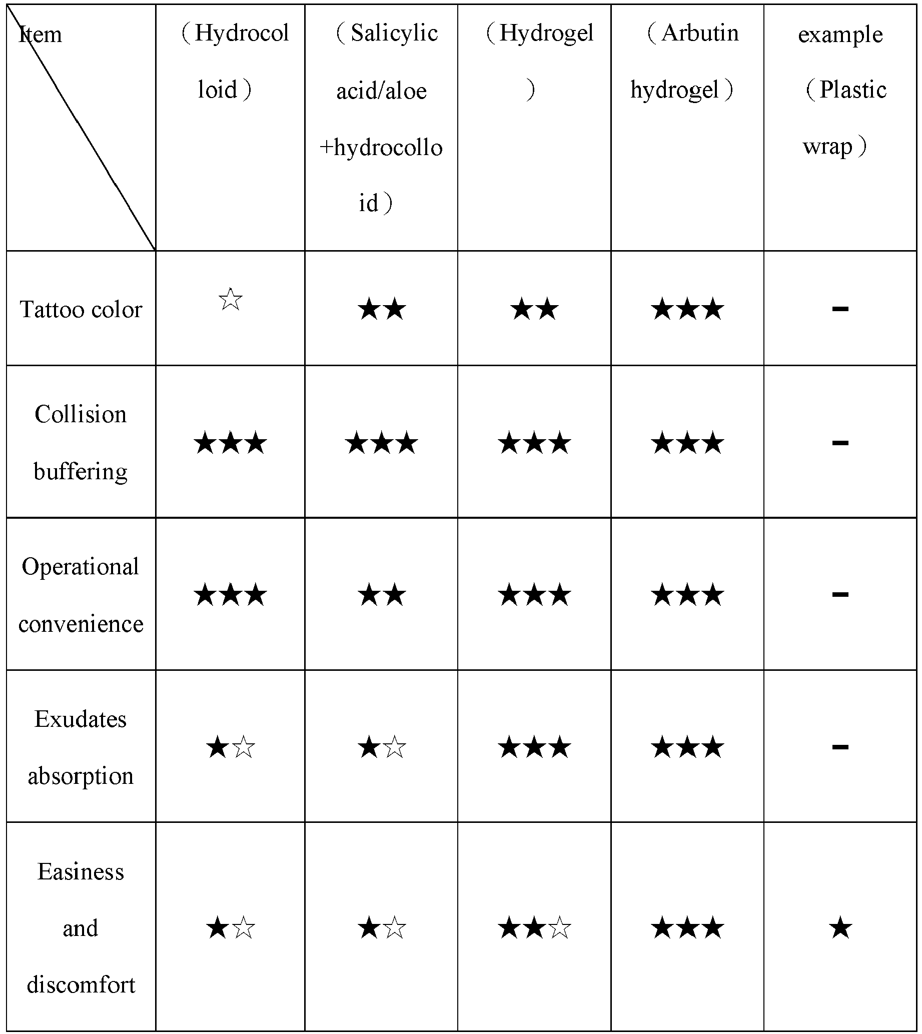

The examples above are merely exemplary to describe the efficacy of the present invention and to illustrate the technical features of the present invention, and they are not intended to limit the scope of the present invention. One skilled in the art may modify and vary the examples without departing from the spirit and scope of the present invention, therefore, the examples should not be construed as the limitation of the claims.

## Claims

1. A method for maintaining and enhancing colors of a tattoo pattern, comprising:
(a) tattooing a pattern on the skin of a subject; and
(b) covering a moist dressing on the pattern, wherein the moist dressing is used for maintaining bright colors of the pattern and providing a retouching effect;
wherein the moist dressing comprises a solid sheet-like dressing or a liquid spray-on film dressing, wherein the solid sheet-like dressing comprises a transparent film dressing, a hydrogel dressing, a hydrocolloid dressing, a hydrofiber dressing, an alginate dressing, a silicone dressing or a chitosan dressing.

2. The method according to claim 1, wherein the moist dressing comprises a soothing factor, a retouching factor or a combination thereof.

3. The method according to claim 2, wherein the soothing factor is selected from the group consisting of collagen, hyaluronic acid, glycerin, neuroamide, gelatin, growth factors, cytokines, antibacterial peptides, chitosan, diclofenac sodium, indomethacin, menthol, camphor, thymol, tea tree oil, clove oil, borneol, capsaicin, nicotinic acid benzyl ester, eucalyptus oil, curcumin, musk, cinnamon, angelica, frankincense and derivatives thereof.

4. The method according to claim 2, wherein the retouching factor is selected from the group consisting of glycyrretinic acid, salvia miltiorrhiza, allantoin, aloe, arbutin, vitamin C (ascorbyl), salicylic acid, ellagic acid, kojic acid, chamomile extract (charmomile ET), tranexamic acid, 5,5'-dipropyl-biphenyl-2,2'-diol and dervatives thereof.

5. The method according to claim 2, wherein the weight percentage of the soothing factor or retouching factor is from 0.01% to 10% by weight of the total components of the moist dressing.

6. The method according to claim 2, wherein the retouching factor comprises potassium methoxysalicylate, aloe or a combination thereof.

7. The method according to claim 2, wherein the retouching factor comprises arbutin, and the soothing factor comprises hyaluronic acid.

8. The method according to claim 1, wherein the moist dressing has a waterproof and air-permeable function.
